(19) Europäisches Patentamt
European Patent Office
Office européen des brevets

(11) **EP 3 142 073 A1**

(12) **EUROPÄISCHE PATENTANMELDUNG**

(43) Veröffentlichungstag:
15.03.2017 Patentblatt 2017/11

(51) Int Cl.:
*G06T 7/73* (2017.01)   *A61B 6/12* (2006.01)

(21) Anmeldenummer: 16183384.3

(22) Anmeldetag: 09.08.2016

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(30) Priorität: **08.09.2015 DE 102015115060**

(71) Anmelder: **BIOTRONIK SE & Co. KG**
**12359 Berlin (DE)**

(72) Erfinder: **Rump, Jens**
**12049 Berlin (DE)**

(74) Vertreter: **Galander, Marcus**
**Biotronik SE & Co. KG**
**Corporate Intellectual Properties**
**Woermannkehre 1**
**12359 Berlin (DE)**

(54) **VERFAHREN, COMPUTERPROGRAMM UND SYSTEM ZUM BESTIMMEN DES RÄUMLICHEN VERLAUFS EINES KÖRPERS**

(57) Die Erfindung betrifft ein Verfahren zur Rekonstruktion des räumlichen Verlaufs eines längs erstreckten, flexiblen Körpers (1) in einem 3D-Weltkoordinatensystem, wobei der Körper (1) eine Mehrzahl an Röntgenmarkern (10) aufweist, die beabstandet zueinander entlang des Körpers (1) an diesem angeordnet sind, aufweisend die Schritte: Bereitstellen eines 2D-Röntgenbildes des Körpers (1), Bestimmen der zweidimensionalen Positionen der Röntgenmarker (10) in einem Bildkoordinatensystem des 2D-Röntgenbildes (B), Bestimmen von möglichen 3D-Ortskoordinaten jedes Röntgenmarkers (10) in dem 3D-Weltkoordinatensystem als Strahlen, die jeweils von einem Ursprung, der der Position der Strahlenquelle zur Erzeugung des 2D-Röntgenbildes entspricht, zur Position des jeweiligen Röntgenmarkers (10) in einer Bildkoordinatenebene verlaufen, und iterative Bestimmung des räumlichen Verlaufs des Körpers (1) unter Verwendung der besagten möglichen 3D-Ortskoordinaten. Weiterhin betrifft die Erfindung ein entsprechendes Computerprogramm sowie ein entsprechendes System.

Fig. 1

EP 3 142 073 A1

**Beschreibung**

Die Erfindung betrifft ein Verfahren, ein Computerprogramm und ein System zur Rekonstruktion des räumlichen Verlaufs einer längs erstreckten, flexiblen Elektrode in einem 3D-Weltkoordinatensystem.

Entscheidend für die erfolgreiche Implantation von Elektroden (z. B. für Herzschrittmacher) ist die optimale Platzierung der Elektrode innerhalb des Herzens. Aus diesem Grund wird die Implantation im Allgemeinen von Echtzeit-Fluoroskopie-Aufnahmen mittels Röntgenbildgebung überwacht. Bei diesen Aufnahmen ist die Elektrodenlage innerhalb des Herzens allerdings nur aus einer einzigen Ansicht als Durchleuchtungsbild erhältlich. Für die Kontrolle der Elektrodenlage aus einem anderen Blickwinkel muss das Bildgebungsgerät neu justiert werden. Geräte, die gleichzeitig Bildgebung aus mehr als einer Blickrichtung ermöglichen (biplanares Röntgen, CT, MRT) sind für die interoperative Bildgebung oft nicht verfügbar oder ungeeignet. Zudem erhöht sich bei biplanarem Röntgen oder CT die Strahlenbelastung für den Arzt und Patienten erheblich. Weiterhin sind aktive 3D-Trackingmethoden oftmals kostspielig und üblicherweise nur im Forschungsbereich verfügbar.

Der Erfindung liegt hiervon ausgehend die Aufgabe zugrunde, ein Verfahren, ein Computerprogramm und ein System zur Rekonstruktion des räumlichen Verlaufs eines Körpers, insbesondere einer Elektrode, bereitzustellen, das die vorgenannten Nachteile zumindest teilweise beseitigt.

Dieses Problem wird durch ein Verfahren gemäß Anspruch 1, ein Computerprogramm gemäß Anspruch 6 sowie durch ein System gemäß Anspruch 9 gelöst. Vorteilhafte Ausgestaltungen dieser Aspekte der Erfindung sind in den jeweils zugehörigen Unteransprüchen angegeben und werden nachfolgend beschrieben.

Gemäß Anspruch 1 ist ein Verfahren zur Rekonstruktion des räumlichen Verlaufs eines längserstreckten, flexiblen Körpers in einem 3D-Weltkoordinatensystem vorgesehen, wobei der Körper einer Mehrzahl an Röntgenmarkern aufweist, die beabstandet zueinander entlang des Körpers an diesem angeordnet sind, und wobei das Verfahren die Schritte aufweist:

- Bereitstellen eines 2D-Röntgenbildes des Körpers,
- Bestimmen der zweidimensionalen Positionen der Röntgenmarker in einem 2D-Bildkoordinatensystem des 2D-Röntgenbildes,
- Bestimmen von möglichen 3D-Ortskoordinaten jedes Röntgenmarkers in dem 3D-Weltkoordinatensystem als Strahlen, die jeweils von einem Ursprung, der der Position der Strahlenquelle zur Erzeugung des 2D-Röntgenbildes entspricht, zur Position des jeweiligen Röntgenmarkers in einer Bildkoordinatenebene des Röntgenbildes verlaufen, und
- iterative Bestimmung des räumlichen Verlaufs des Körpers (z. B. in Form einer angefitteten 3D-Kurve) unter Verwendung der besagten möglichen 3D-Ortskoordinaten.

Röntgenmarker bedeutet hier, dass diese Marker in hinreichendem Maße undurchlässig für Röntgenstrahlen sind, so dass Sie in einem Röntgenbild einen detektierbaren Kontrast bilden.

Mit anderen Worten ermöglicht die vorliegende Erfindung also durch eine gleichmäßige Anordnung von Röntgenmarkern entlang des Körpers bzw. der Elektrode den Erhalt zusätzlicher Informationen über die 3D-Position des Körpers bzw. der Elektrode, und zwar über die projizierte Geometrie der Marker, so dass die Möglichkeit gegeben ist nur auf Grundlage einer 2D-Bildgebung die 3D-Lage des Körpers bzw. der Elektrode zu rekonstruieren.

Gemäß einer Ausführungsform des erfindungsgemäßen Verfahrens ist - wie bereits angedeutet - vorgesehen, dass der Körper eine Elektrode aufweist oder als eine Elektrode ausgebildet ist. Diese ist vorzugsweise flexibel, also biegbar, sowie bevorzugt längserstreckt ausgebildet. Letzteres bedeutet, dass der Körper bzw. die Elektrode entlang einer Längsachse, entlang der er bzw. sie sich von einem distalen Ende zu einem proximalen Ende erstreckt, eine größere Ausdehnung aufweist als senkrecht zur Längsachse.

Weiterhin ist gemäß einer bevorzugten Ausführungsform des erfindungsgemäßen Verfahrens vorgesehen, dass die besagte iterative Bestimmung folgende Schritte aufweist:

(a) vorzugsweise Vorgeben von Startwerten für die 3D-Ortskoordinaten aus der Menge der möglichen 3D-Ortskoordinaten (z. B. auf einer Ebene mittig zwischen der Strahlenquelle und einem Detektor bzw. der Bildkoordinatenebene parallel zum Detektor bzw. der Bildkoordinatenebene, und zwar je Röntgenmarker ein Punkt jener Ebene) und Anfitten einer 3D-Kurve an die so erhaltenen anfänglichen 3D-Ortskoordinaten der Röntgenmarker im Weltkoordinatensystem,

(b) Verschieben zumindest einer 3D-Ortskoordinate entlang des zugeordneten Strahls auf eine mögliche weitere 3D-Ortskoordinate, um aktualisierte 3D-Ortskoordinaten zu erhalten,

(c) Anfitten einer 3D-Kurve an die aktualisierten 3D-Ortskoordinaten,

(d) Rückprojektion der 3D-Kurve in das Bildkoordinatensystem (auf die 2D-Bildkoordinaten) und Vergleich der Rückprojektion mit dem 2D-Röntgenbild, insbesondere hinsichtlich der Größe, Orientierung und/oder Position der Röntgenmarker im Röntgenbild,

(e) Fortsetzen der iterativen Bestimmung beginnend mit Schritt (b) bis zum Erreichen eines vordefinierten Kriteriums

Nach Abgleich dieser Projektion und der tatsächlichen Lage der Elektrode im Röntgenbild, wird die Ortsposition (3D-Ortskoordinaten) der Marker vorzugsweise entsprechend verändert und durch übliche Optimierungsalgorithmen (z. B. Gradientenverfahren, Particle-Swarm Optimization, Genetic Algorithm) die wahrscheinlichste Lage ermittelt. Die Abbruchkriterien für die Optimierung ergeben sich aus der angestrebten Genauigkeit der Rekonstruktion. Das theoretische Minimum der Optimierung ist dabei eine Differenz aus Rückprojektion und 2D-Röntgenbild, die im Bereich der Bildauflösung des Röntgenbildes liegt. Dabei ist zu berücksichtigen, dass sich die Dauer des Optimierungsvorgangs mit erhöhter angestrebter Genauigkeit erhöht.

Vorzugsweise ist die Zielgröße bzw. sind die Zielgrößen, die durch die Optimierung minimiert werden die Abweichungen der Rückprojektion der einzelnen Komponenten des Objektes bzw. Hülsen zum 2D-Röntgenbild. Ist die Rückprojektion im Vergleich zum 2D-Röntgenbild zu groß, werden die Ortskoordinaten vorzugsweise in Richtung der Bildkoordinatenebene verschoben. Sind sie zu klein, werden sie vorzugsweise in Richtung der Strahlenquellen verschoben. Die genaue Systematik, nach der die Koordinaten der 3D-Ortskoordinaten verschoben werden, kann vom gewählten Algorithmus der Optimierung abhängen bzw. wird durch das gewählte Optimierungsverfahren (siehe oben) festgelegt.

Da der Abstand benachbarter Hülsen zueinander klar definiert ist (dieser wird auf einen linear erstreckten Körper bezogen) und die maximale Krümmung aufgrund der elastischen Eigenschaften und dem Durchmesser des Körpers bzw. der Elektrode eingegrenzt werden kann, ergibt sich für jede mögliche Position einer Hülse n auf der entsprechenden Gerade bzw. dem entsprechenden Strahl für die mögliche Position der angrenzenden Hülse n+1 eine Kugelschale.

Gemäß einer bevorzugten Ausführungsform des erfindungsgemäßen Verfahrens ist daher weiterhin vorgesehen, dass im oben aufgeführten Schritt (b) die weitere Ortskoordinate so gewählt wird, dass sie auf dem der weiteren Ortskoordinate zugeordneten Strahl sowie in einer Kugelschale um eine 3D-Ortskoordinate eines benachbarten Röntgenmarkers liegt, wobei der äußere Radius der Kugelschale durch den Abstand der beiden benachbarten Röntgenmarker entlang des Körpers gegeben ist, und wobei der innere Radius der Kugelschale durch die Länge einer zwischen den beiden Röntgenmarkern erstreckte Bogensehne bei maximaler Krümmung des Körpers zwischen den beiden benachbarten Röntgenmarkern gegeben ist.

Vorzugsweise ist der äußere Radius gleich dem Abstand der betreffenden benachbarten Hülsen: $R_{außen}=D$, der auf einen linearen Verlauf des Körpers bezogen ist.

Vorzugsweise ist der innere Radius der Kugelschale dabei durch die Länge der Bogensehne bei maximaler Krümmung r gegeben:

$$\text{ne bei maximaler Krümmung r gegeben: } R_{innen}=2*r*\sin(D/(2*r)).$$

Die Geradenabschnitte bzw. Strahlenabschnitte, die sich aus dem Schnitt der Kugelschale mit den Strahlen des Röntgenmarkers n+1 ergeben, schränken die möglichen 3D Positionen der Marker für die Optimierung der Körper- bzw. Elektrodenlage auf maximal zwei Bereiche innerhalb der Kugelschale ein.

Die Information, welcher der beiden Bereiche für die Position der Hülse infrage kommt, kann aus der parallaktischen Vergrößerung der Hülse geschlossen werden. Zusätzliche Informationen liefert die Winkelung und geometrische Verkürzung des jeweiligen Markers bzw. der jeweiligen Hülse in der Detektorebene bzw. der Bildkoordinatenebene. Das räumliche Auflösungsvermögen außerhalb der Bildachse hängt vornehmlich vom Auflösungsvermögen des Röntgenbildes ab.

Weiterhin ist gemäß einer bevorzugten Ausführungsform des erfindungsgemäßen Verfahrens vorgesehen, dass die Röntgenmarker ringförmig ausgebildet sind, und zwar insbesondere als Hülsen, wobei jene Hülsen z. B. metallische Hülsen sein können.

Gemäß einer bevorzugten Ausführungsform des erfindungsgemäßen Verfahrens ist weiterhin vorgesehen, dass benachbarte Röntgenmarker in Abständen von 1 cm bis 5 cm, bevorzugt 2 cm, zueinander angeordnet sind. Insbesondere können die (z. B. metallischen) Hülsen einen Außendurchmesser von 2 mm sowie eine Länge von 1mm entlang der Längsachse des Körpers bzw. der Elektrode aufweisen. Der Abstand 2 cm ergibt sich insbesondere z. B. aus der Annahme, dass von durchschnittlichen Krümmungsradien der Elektrode im intrakardialen Bereich von um die r=2 cm (Krümmung: 0.5 1/cm) auszugehen ist. Die Wandstärke der Hülsen sollte für einen sichtbaren Röntgenkontrast ausreichen und ist gemäß einer Ausführungsform größer gleich 0,05 mm.

Weiterhin ist gemäß einer bevorzugten Ausführungsform des erfindungsgemäßen Verfahrens vorgesehen, dass die Röntgenmarker metallische Partikel aufweisen, die in Ringform in eine Isolierung des Körpers bzw. der Elektrode eingebracht sind.

Weiterhin ist gemäß einer bevorzugten Ausführungsform des erfindungsgemäßen Verfahrens vorgesehen, dass die Röntgenmarker ein metallisches Geflecht aufweisen, das in einer Isolierung des Körpers bzw. der Elektrode angeordnet ist.

Weiterhin ist gemäß einer bevorzugten Ausführungsform des erfindungsgemäßen Verfahrens vorgesehen, dass die Röntgenmarker sich hinsichtlich ihrer räumlichen Dimensionen untereinander unterscheiden, insbesondere in Abhängigkeit von Ihrer Position entlang des Körpers bzw. der Elektrode, insbesondere derart, dass die entsprechenden Kontraste der Röntgenmarker im 2D-Röntgenbild unterscheidbar sind.

Gemäß einem weiteren Aspekt der Erfindung wird ein Computerprogramm zur Rekonstruktion des räumlichen Verlaufs eines längserstreckten, flexiblen Körpers in einem 3D-Weltkoordinatensystem offenbart, wobei der Körper einer Mehrzahl an Röntgenmarkern aufweist, die beabstandet zueinander entlang des Körpers an diesem angeordnet sind, und wobei das Computerprogramm einen Programmcode aufweist, der dazu ausgebildet ist, die nachfolgenden Schritte auszuführen, wenn das Computerprogramm auf einem Computer ausgeführt wird:

- Bestimmen der zweidimensionalen Positionen der Röntgenmarker in einem 2D-Bildkoordinatensystem eines von dem Körper aufgenommenen 2D-Röntgenbildes,
- Bestimmen von möglichen 3D-Ortskoordinaten jedes Röntgenmarkers in dem 3D-Weltkoordinatensystem als Strahlen, die jeweils von einem Ursprung, der der Position der Strahlenquelle zur Erzeugung des 2D-Röntgenbildes entspricht, zur Position des jeweiligen Röntgenmarkers in einer Bildkoordinatenebene verlaufen, und
- iterative Bestimmung des räumlichen Verlaufs des Körpers unter Verwendung der besagten möglichen 3D-Ortskoordinaten.

Gemäß einer bevorzugten Ausführungsform des erfindungsgemäßen Computerprogramms ist wiederum vorgesehen, dass der Körper eine Elektrode aufweist oder als Elektrode ausgebildet ist (siehe auch oben).

Weiterhin ist wiederum gemäß einer Ausführungsform des erfindungsgemäßen Computerprogramms vorgesehen, dass die besagte iterative Bestimmung folgende Schritte aufweist:

(a) Anfitten einer 3D-Kurve an vorgegebene Startwerte für die 3D-Ortskoordinaten der Röntgenmarker (Weltkoordinatensystem) aus der Menge der möglichen 3D-Ortskoordinaten,
(b) Verschieben zumindest einer 3D-Ortskoordinate der 3D-Kurve entlang des zugeordneten Strahls auf eine mögliche weitere 3D-Ortskoordinate,
(c) Anfitten einer 3D-Kurve an die solchermaßen aktualisierten 3D-Ortskoordinaten der 3D-Kurve,
(d) Rückprojektion dieser 3D-Kurve in das Bildkoordinatensystem (auf die 2D-Bildkoordinaten) und Vergleich der Rückprojektion mit dem 2D-Röntgenbild, insbesondere hinsichtlich der Größe, der Orientierung und/oder der Position der Röntgenmarker im Röntgenbild, und
(e) Fortsetzen der iterativen Bestimmung beginnend mit Schritt (b) bis zum Erreichen eines vordefinierten Kriteriums.

Hierzu wird auch auf die obigen Erläuterungen verwiesen.

Gemäß einer bevorzugten Ausführungsform des erfindungsgenäßen Computerprogramms wird wiederum - wie bereits oben dargelegt - im Schritt (b) die weitere Ortskoordinate so gewählt, dass sie auf dem der weiteren Ortskoordinate zugeordneten Strahl sowie in einer Kugelschale um eine 3D-Ortskoordinate eines benachbarten Röntgenmarkers liegt, wobei der äußere Radius der Kugelschale durch den Abstand der beiden benachbarten Röntgenmarker entlang des Körpers gegeben ist, und wobei der innere Radius der Kugelschale durch die Länge einer zwischen den beiden Röntgenmarkern erstreckte Bogensehne bei maximaler Krümmung des Körpers zwischen den beiden benachbarten Röntgenmarkern gegeben ist.

Die Röntgenmarker sind wiederum vorzugsweise in einer der oben bereits beschriebenen Arten ausgebildet und zueinander angeordnet.

Gemäß einem weiteren Aspekt der vorliegenden Erfindung wird ein System zur Rekonstruktion des räumlichen Verlaufs eines längs erstreckten, flexiblen Körpers in einem 3D-Weltkoordinatensystem vorgeschlagen, wobei das System zumindest aufweist: einen längserstreckten, flexiblen sowie implantierbaren Körper, der gemäß einer Ausführungsform des Systems vorzugsweise als eine Elektrode ausgebildet ist oder vorzugsweise eine Elektrode aufweist, und eine Mehrzahl an Röntgenmarkern, die beabstandet zueinander entlang des Körpers am Körper angeordnet sind.

Bevorzugt sind wiederum - wie bereits oben erläutert - die Röntgenmarker ringförmig ausgebildet, und zwar bevorzugt als Hülsen, wobei vorzugsweise benachbarte Röntgenmarker in Abständen von 1 cm bis 5 cm, bevorzugt 2 cm, zueinander angeordnet sind. Vorzugsweise weisen alle benachbarten Röntgenmarker die gleichen Abstände (z. B. D=2 cm) auf. Die Längen der Hülsen können je nach Position variieren. Dies gilt auch für das hierin beschriebene Verfahren bzw. Computerprogramm.

Gemäß einer bevorzugten Ausführungsform des erfindungsgemäßen Systems ist vorgesehen, dass die Röntgenmarker metallische Partikel aufweisen, die in Ringform in eine Isolierung des Körpers eingebracht sind, oder dass die Röntgenmarker jeweils ein metallisches Geflecht aufweisen, das in einer Isolierung des Körpers bzw. der Elektrode angeordnet ist (vergleiche auch oben).

Gemäß einer bevorzugten Ausführungsform des erfindungsgemäßen Systems ist weiterhin vorgesehen, dass die Röntgenmarker sich hinsichtlich ihrer räumlichen Dimensionen untereinander unterscheiden, insbesondere in Abhängigkeit von Ihrer Position entlang der Körpers, insbesondere so, dass die entsprechenden Kontraste der Röntgenmarker im 2D-Röntgenbild unterscheidbar sind (siehe auch oben).

Einer bevorzugte Ausführungsform des erfindungsgemäßen Systems ist weiterhin, dass das System eine Röntgeneinrichtung aufweist, die dazu konfiguriert ist, ein 2D-Röntgenbild des Körpers zu erzeugen, sowie ein Analysemittel, das dazu konfiguriert ist, die zweidimensionalen Positionen der Röntgenmarker in einem 2D-Bildkoordinatensystem des 2D-Röntgenbildes zu bestimmen, und das weiterhin dazu konfiguriert ist, mögliche 3D-Ortskoordinaten jedes Röntgenmarkers in dem 3D-Weltkoordinatensystem als Strahlen zu bestimmen, die jeweils von einem Ursprung, der der Position der Strahlenquelle zur Erzeugung des 2D-Röntgenbildes entspricht, zur Position des jeweiligen Röntgenmarkers in einer Bildkoordinatenebene verlaufen, und das ferner dazu konfiguriert ist, den räumlichen Verlaufs des Körpers unter Verwendung der besagten möglichen 3D-Ortskoordinaten iterativ zu bestimmen.

Das Analysemittel kann einen Computer (z. B. handelsüblichen PC) aufweisen, auf dem eine geeignete Software (z. B. das erfindungsgemäße Computerprogramm) ausgeführt wird bzw. ausführbar ist. Das Analysemittel kann aber auch in anderer Weise ausgebildet sein (z. B. als reine Hardwarelösung mit fest integrierter Software).

Gemäß einer bevorzugten Ausführungsform des erfindungsgemäßen Systems ist weiterhin vorgesehen, dass das Analysemittel dazu konfiguriert ist, zur besagten iterative Bestimmung:

(a) an vorgegebene Startwerte für die 3D-Ortskoordinaten aus der Menge der möglichen 3D-Ortskoordinaten (z. B. auf der Ebene mittig zwischen Strahlenquelle und einem Detektor der Röntgeneinrichtung parallel zum Detektor bzw. Bildkoordinatenebene (je Röntgenmarker ein Punkt im Raum) eine 3D-Kurve anzufitten,
(b) zum Erhalt aktualisierter 3D-Ortskoordinaten zumindest eine 3D-Ortskoordinate der 3D-Kurve entlang des zugeordneten Strahls auf eine mögliche weitere 3D-Ortskoordinate zu verschieben,
(c) eine 3D-Kurve an die aktualisierten 3D-Ortskoordinaten anzufitten,
(d) eine Rückprojektion der 3D-Kurve zu den aktualisierten 3D-Koordinaten in das Bildkoordinatensystem (auf die 2D-Bildkoordinaten) vorzunehmen und jene Rückprojektion mit dem 2D-Röntgenbild zu vergleichen, und zwar insbesondere hinsichtlich der Größe, der Orientierung und der Position der Röntgenmarker im Röntgenbild, und
(e) die iterative Bestimmung beginnend mit Schritt (b) bis zum Erreichen eines vordefinierten Kriteriums fortzusetzen.

Gemäß einer bevorzugten Ausführungsform des erfindungsgemäßen Systems ist weiterhin vorgesehen, dass das Analysemittel dazu ausgebildet ist, im Schritt (b) die weitere Ortskoordinate so zu wählen, dass sie auf dem der weiteren Ortskoordinate zugeordneten Strahl sowie in einer Kugelschale um eine 3D-Ortskoordinate eines benachbarten Röntgenmarkers liegt, wobei der äußere Radius der Kugelschale durch den Abstand der beiden benachbarten Röntgenmarker entlang des Körpers gegeben ist, und wobei der innere Radius der Kugelschale durch die Länge einer zwischen den beiden Röntgenmarkern erstreckte Bogensehne bei maximaler Krümmung des Körpers zwischen den beiden benachbarten Röntgenmarkern gegeben ist (hierzu wird auch auf die obigen Erläuterungen verwiesen).

Weitere Merkmale und Vorteile der Erfindung sollen bei der Figurenbeschreibung eines Ausführungsbeispiels der Erfindung anhand der Figuren erläutert werden. Es zeigen:

Fig. 1    eine schematische Darstellung eines erfindungsgemäßen Körpers in Form einer Elektrode mit Röntgenmarkern, die entlang des Körpers angeordnet sind und dabei zueinander beabstandet sind;

Fig. 2    eine schematische Darstellung der Erzeugung eines 2D Röntgenbildes der Röntgenmarker;

Fig. 3    ein Ablauf einer Ausführungsform eines erfindungsgemäßen Verfahrens bzw. Computerprogramms zur 3D-Rekonstuktion einer Elektrode anhand eines 2D Röntgenbildes;

Fig. 4    eine schematische Darstellung der 3D-Rekonstruktion eines Elektrodenverlaufs bei einem erfindungsgemäßen Verfahren oder Computerprogramm; und

Fig. 5    eine Illustration von physikalischen Restriktionen bei der Verschiebung einer 3D-Ortskoordinaten auf einem Strahl möglicher 3D-Ortskoordinaten.

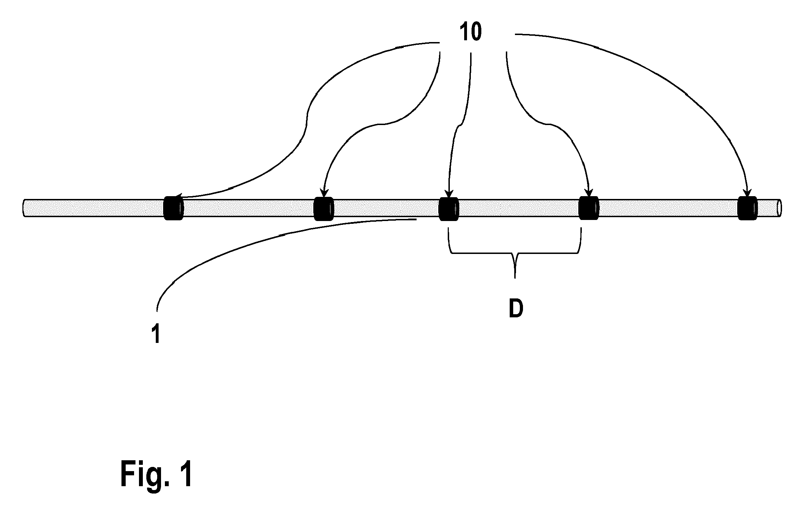

Figur 1 zeigt einen erfindungsgemäßen Körper 1 in Form einer Elektrode, insbesondere Brady-Elektrode, mit einem Außendurchmesser von z. B. 2 mm (z. B. Siello). Die Elektrode 1 erstreckt sich längs einer Längsachse und weist Röntgenmarker 10 in Form von metallischen Hülsen auf (z. B. MP35N), wobei benachbarte Hülsen zueinander einen Abstand von D=2 cm aufweisen. Die Hülsen 10 weisen weiterhin beispielsweise einen Außendurchmesser von 2 mm auf sowie z. B. eine Länge von 1 mm (entlang der Längsachse der Elektrode). Der Abstand 2 cm ergibt sich insbesondere aus der Annahme, dass von durchschnittlichen Krümmungsradien einer Elektrode 1 im intrakardialen Bereich von um die r=2 cm (Krümmung: 0.5 1/cm) auszugehen ist. Die Wandstärke der Hülsen 10 erzeugt bevorzugt einen sichtbaren Röntgenkontrast und ist z. B. größer gleich 0,05 mm.

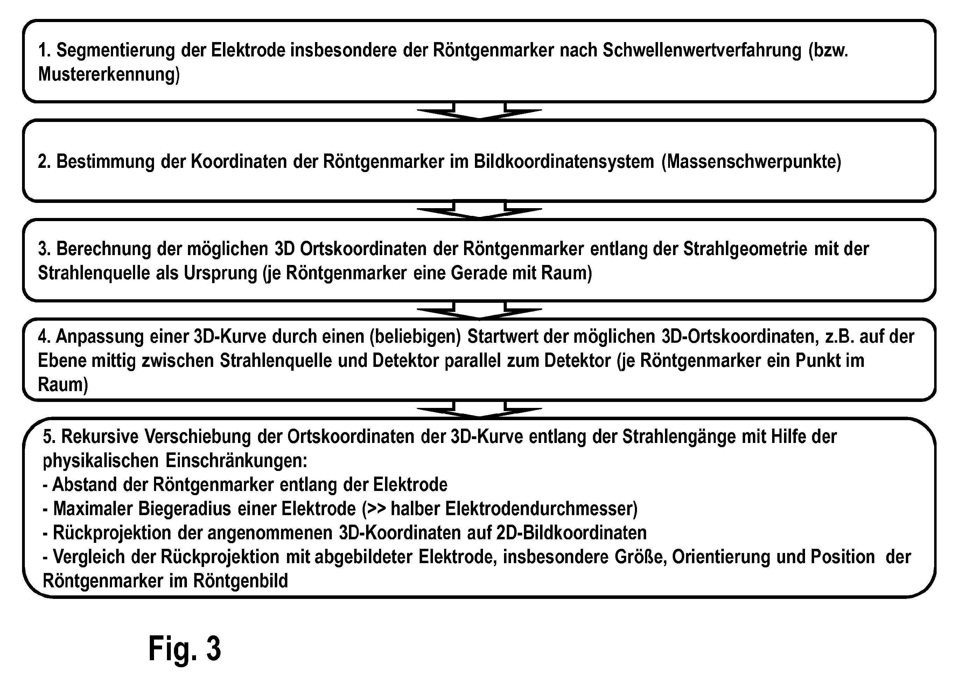

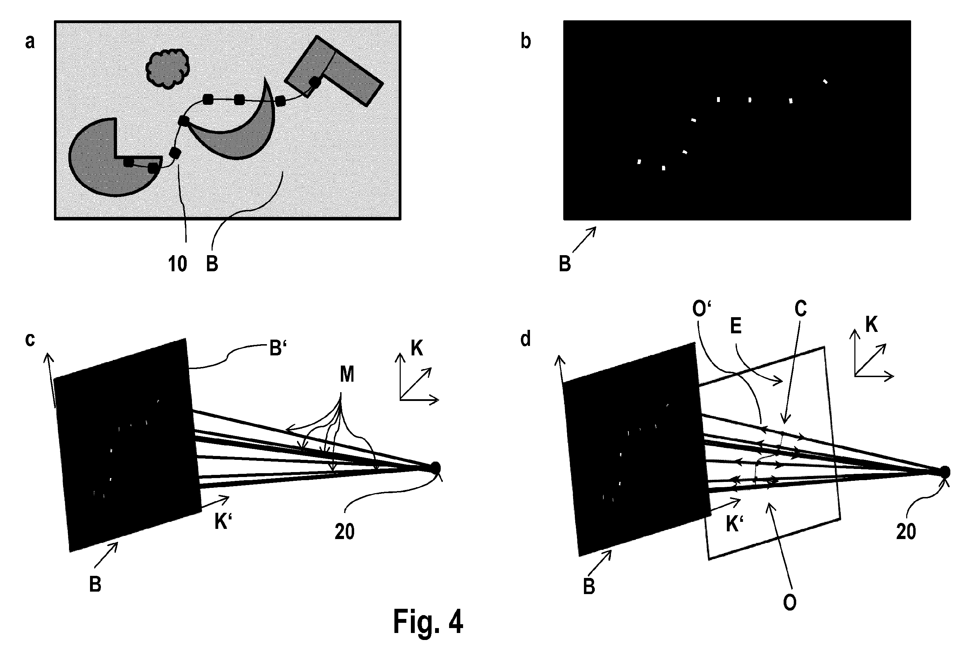

Ein möglicher Ablauf der 3D-Rekonstruktion der Elektrode 1 ist exemplarisch in der Figur 3 dargestellt. Vorzugsweise mittels (automatischer) Mustererkennung werden die Positionen der Hülsen 10 im 2D-Röntgenbild B (vgl. Figur 4 a) bestimmt (vgl. Figur 4 b). Je nach Röntgen-Kontrast reicht dafür eine Umwandlung des Röntgenbildes in ein Binärbild nach Schwellwertbildung aus. Der Kontrast kann optional durch den Phasenkontrast und/oder Kantenkontrastverstärkung (z. B. Canny-, Sobel-, Prewitt-Filter) erhöht werden.

Nach einer Segmentierung des Röntgenbildes B kann die Position der Röntgenmarker 10 in Bildkoordinaten (z. B. durch Bestimmung der Massenschwerpunkte innerhalb des Binärbildes B) ermittelt werden. Die Position in Bildkoordinaten führt nach Anwendung der Strahlengeometrie zu möglichen Positionen M der Hülsen 10 im 3D-Raum K entlang von Geraden bzw. Strahlen M mit der Position der Strahlenquelle 20 als Ursprung (Abbildung 4 c).

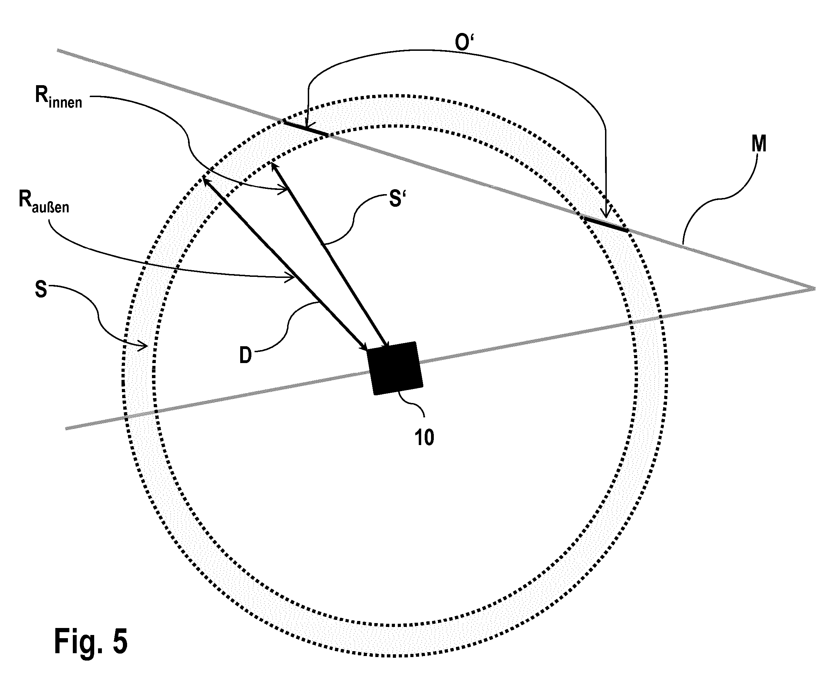

Da der Abstand der Hülsen 10 klar definiert ist und die maximale Krümmung aufgrund der elastischen Eigenschaften und dem Durchmesser einer Elektrode 1 eingegrenzt werden kann, ergibt sich für jede mögliche Position einer n-ten Hülse 10 auf der entsprechenden Gerade M für die mögliche Position der angrenzenden (n+1) Hülse 10 eine Kugelschale S (vgl. Fig. 5).

Der äußere Radius $R_{außen}$ der Kugelschale S ist gleich dem Abstand D der benachbarten Hülsen: $R_{außen}$=D. Der innere Radius $R_{innen}$ der Kugelschale S ist durch die Länge der Bogensehne S' bei maximaler Krümmung r gegeben: $R_{innen}$=2*r*sin(D/(2*r)).

Die Geradenabschnitte O', die sich aus dem Schnitt der Kugelschale S mit dem Strahl M der benachbarten (n+1) Hülse 10 ergeben, schränken die möglichen 3D-Positionen der Hülsen 10 für die Optimierung der Elektrodenlage stark ein.

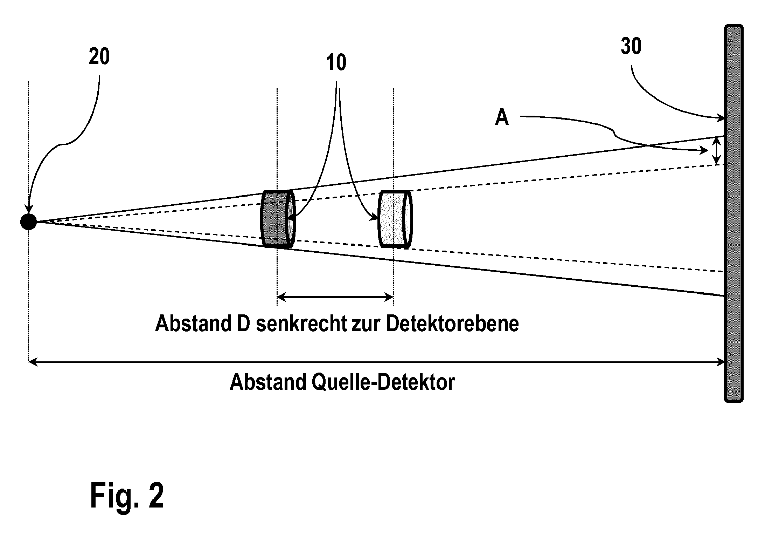

Die Information, ob die Position der Hülse 10 ausgehend von der Position der Strahlenquelle 20 auf dem vorderen oder hinteren Geradenabschnitt liegt, kann aus der parallaktischen Vergrößerung der Hülse geschlossen werden (vgl. Figur 2). Zusätzliche Informationen liefert die Winkelung und geometrische Verkürzung der Hülse 10 in der Detektorebene des Detektors 30. Das räumliche Auflösungsvermögen außerhalb der Bildachse hängt vornehmlich vom Auflösungsvermögen des Röntgenbildes B ab.

Bei einem typischen Abstand von Strahlungsquelle 20 zu Strahlendetektor 30 von 1m und einer angenommenen Entfernung der Elektrode zur Strahlungsquelle von circa 0,5 m, beträgt die räumliche Tiefenauflösung bei einer typischen Bildauflösung des Detektors 30 von 0,2 mm etwa 2,6 cm.

Die Bestimmung der 3D-Koordinaten der Elektrode 1 erfolgt vorzugsweise durch rekursive Bestimmung der 3D-Ortskoordinaten der Röntgenmarker 10 ausgehend von einem Startwert E und Kurven-Fitting (z. B. Non-Negative-Least-Square NNLS) durch die angenommenen Positionen der Hülsen. Für das Kurvenfitting wird vorzugsweise ein Polynom vierter Ordnung verwendet, da sich damit die elastischen Parameter einer Elektrode 1 am genauesten abbilden lassen.

Bei jedem Iterationsschritt werden die Positionen der Hülse 10 entlang den zugehörigen Ortsgeraden M unter Berücksichtigung der physikalischen Restriktionen (siehe oben, Fig. 5) verschoben, eine 3D Kurve C durch die neuen Ortskoordinaten O (vgl. Fig. 4c) gefittet und diese Kurve C auf das Röntgenbild B projiziert. Nach Abgleich dieser Projektion und der tatsächlichen Lage der Elektrode 1 im Röntgenbild B, wird die Ortsposition der Marker 10 entsprechend verändert und durch übliche Optimierungsalgorithmen (z. B. Gradientenverfahren, Particle-Swarm Optimization, Genetic Algorithm) die wahrscheinlichste Lage ermittelt. Ist die Abbildung der Hülsen bei der Rückprojektion kleiner als die Größe der Abbildung im 2D-Röntenbild, wird die Position der Hülse in Richtung der Strahlenquelle verschoben. Ist die Abbildung der Hülsen bei der Rückprojektion größer als die Größe der Abbildung im 2D-Röntenbild, wird die Position der Hülse in Richtung der Bildkoordinatenebene verschoben.

**Patentansprüche**

1. Verfahren zur Rekonstruktion des räumlichen Verlaufs eines längs erstreckten, flexiblen Körpers (1) in einem 3D-Weltkoordinatensystem (K), wobei der Körper (1) eine Mehrzahl an Röntgenmarkern (10) aufweist, die beabstandet zueinander entlang des Körpers (1) an diesem angeordnet sind, aufweisend die Schritte:

   - Bereitstellen eines 2D-Röntgenbildes (B) des Körpers (1),
   - Bestimmen der zweidimensionalen Positionen der Röntgenmarker (10) in einem Bildkoordinatensystem (K')
     des 2D-Röntgenbildes (B),

- Bestimmen von möglichen 3D-Ortskoordinaten (M) jedes Röntgenmarkers (10) in dem 3D-Weltkoordinatensystem (K) als Strahlen (M), die jeweils von einem Ursprung, der der Position der Strahlenquelle (20) zur Erzeugung des 2D-Röntgenbildes (B) entspricht, zur Position des jeweiligen Röntgenmarkers (10) in einer Bildkoordinatenebene (B') verlaufen, und

- iterative Bestimmung des räumlichen Verlaufs des Körpers (1) unter Verwendung der besagten möglichen 3D-Ortskoordinaten (M).

**2.** Verfahren nach Anspruch 1, **dadurch gekennzeichnet, dass** der Körper (1) eine Elektrode aufweist oder als Elektrode ausgebildet ist.

**3.** Verfahren nach Anspruch 1 oder 2, **dadurch gekennzeichnet, dass** die besagte iterative Bestimmung folgende Schritte aufweist:

(a) Vorgeben von Startwerten (E) für die 3D-Ortskoordinaten der Röntgenmarker in dem Weltkoordinatensystem (K) aus der Menge der möglichen 3D-Ortskoordinaten (M) und Anfitten einer 3D-Kurve an jene Startwerte (E),
(b) Verschieben zumindest einer 3D-Ortskoordinate entlang des zugeordneten Strahls (M) auf eine mögliche weitere 3D-Ortskoordinate (O'), um aktualisierte 3D-Ortskoordinaten (O) der Röntgenmarker (10) in dem Weltkoordinatensystem (K) zu erhalten,
(c) Anfitten einer 3D-Kurve (C) an die aktualisierten 3D-Ortskoordinaten (O),
(d) Rückprojektion der 3D-Kurve (C) in das Bildkoordinatensystem (K') und Vergleich der Rückprojektion mit dem 2D-Röntgenbild (B), und
(e) Fortsetzen der iterativen Bestimmung beginnend mit Schritt (b) bis zum Erreichen eines vordefinierten Kriteriums.

**4.** Verfahren nach Anspruch 3, **dadurch gekennzeichnet, dass** im Schritt (b) die weitere Ortskoordinate (O') so gewählt wird, dass sie auf dem der weiteren Ortskoordinate zugeordneten Strahl (M) sowie in einer Kugelschale (S) um eine 3D-Ortskoordinate eines benachbarten Röntgenmarkers (10) liegt, wobei der äußere Radius ($R_{außen}$) der Kugelschale (S) durch den Abstand (D) der beiden benachbarten Röntgenmarker (10) entlang des Körpers (1) gegeben ist, und wobei der innere Radius ($R_{innen}$) der Kugelschale (S) durch die Länge einer zwischen den beiden Röntgenmarkern erstreckten Bogensehne (S') bei maximaler Krümmung des Körpers (1) zwischen den beiden benachbarten Röntgenmarkern (10) gegeben ist.

**5.** Verfahren nach einem der vorhergehenden Ansprüche, **dadurch gekennzeichnet, dass** die Röntgenmarker (10) ringförmig ausgebildet sind, insbesondere als Hülsen.

**6.** Computerprogramm zur Rekonstruktion des räumlichen Verlaufs eines längs erstreckten, flexiblen Körpers (1) in einem 3D-Weltkoordinatensystem, wobei der Körper (1) eine Mehrzahl an Röntgenmarkern (10) aufweist, die beabstandet zueinander entlang des Körpers (1) an diesem angeordnet sind, und wobei das Computerprogramm einen Programmcode aufweist, der dazu ausgebildet ist, die nachfolgenden Schritte auszuführen, wenn das Computerprogramm auf einem Computer ausgeführt wird:

- Bestimmen der zweidimensionalen Positionen der Röntgenmarker (10) in einem Bildkoordinatensystem (K') eines von dem Körper (1) aufgenommenen 2D-Röntgenbildes (B),
- Bestimmen von möglichen 3D-Ortskoordinaten (M) jedes Röntgenmarkers (10) in dem 3D-Weltkoordinatensystem (K) als Strahlen, die jeweils von einem Ursprung, der der Position der Strahlenquelle (20) zur Erzeugung des 2D-Röntgenbildes (B) entspricht, zur Position des jeweiligen Röntgenmarkers (10) in einer Bildkoordinatenebene (B') verlaufen, und
- iterative Bestimmung des räumlichen Verlaufs des Körpers (1) unter Verwendung der besagten möglichen 3D-Ortskoordinaten (M).

**7.** Computerprogramm nach Anspruch 6, **dadurch gekennzeichnet, dass** die besagte iterative Bestimmung folgende Schritte aufweist:

(a) Anfitten einer 3D-Kurve an vorgegebene Startwerte für die 3D-Ortskoordinaten der Röntgenmarker (10) in dem Weltkoordinatensystem (K) aus der Menge der möglichen 3D-Ortskoordinaten (M),
(b) Verschieben zumindest einer 3D-Ortskoordinate entlang des zugeordneten Strahls auf eine mögliche weitere 3D-Ortskoordinate, um aktualisierte 3D-Ortskoordinaten (O) der Röntgenmarker (10) in dem Weltkoordinatensystem (K) zu erhalten,

(c) Anfitten einer 3D-Kurve an die aktualisierten 3D-Ortskoordinaten (O),

(d) Rückprojektion der 3D-Kurve (C) in das Bildkoordinatensystem (K') und Vergleich der Rückprojektion mit dem 2D-Röntgenbild (B),

(e) Fortsetzen der iterativen Bestimmung beginnend mit Schritt (b) bis zum Erreichen eines vordefinierten Kriteriums.

**8.** Computerprogramm nach Anspruch 7, **dadurch gekennzeichnet, dass** im Schritt (b) die weitere Ortskoordinate (O') so gewählt wird, dass sie auf dem der weiteren Ortskoordinate (O') zugeordneten Strahl (M) sowie in einer Kugelschale (S) um eine 3D-Ortskoordinate eines benachbarten Röntgenmarkers (10) liegt, wobei der äußere Radius ($R_{außen}$) der Kugelschale (S) durch den Abstand (D) der beiden benachbarten Röntgenmarker (10) entlang des Körpers (1) gegeben ist, und wobei der innere Radius ($R_{innen}$) der Kugelschale (S) durch die Länge einer zwischen den beiden Röntgenmarkern (10) erstreckten Bogensehne (S') bei maximaler Krümmung des Körpers (1) zwischen den beiden benachbarten Röntgenmarkern (10) gegeben ist.

**9.** System zur Rekonstruktion des räumlichen Verlaufs eines längs erstreckten, flexiblen Körpers (1) in einem 3D-Weltkoordinatensystem, mit:

- einem längs erstreckten, flexiblen sowie implantierbaren Körper (1), der vorzugsweise als Elektrode ausgebildet ist oder vorzugsweise eine Elektrode aufweist, und
- einer Mehrzahl an Röntgenmarkern (10), die beabstandet zueinander entlang des Körpers (1) am Körper (1) angeordnet sind.

**10.** System nach Anspruch 9, **dadurch gekennzeichnet, dass** die Röntgenmarker (10) ringförmig ausgebildet sind, und zwar bevorzugt als Hülsen.

**11.** System nach einem der Ansprüche 8 bis 10, **dadurch gekennzeichnet, dass** die Röntgenmarker (10) metallische Partikel aufweisen, die in Ringform in eine Isolierung des Körpers (1) eingebracht sind, oder dass die Röntgenmarker (10) jeweils ein metallisches Geflecht aufweisen, das in einer Isolierung des Körpers (1) angeordnet ist.

**12.** System nach einem der Ansprüche 8 bis 11, **dadurch gekennzeichnet, dass** die Röntgenmarker (10) sich hinsichtlich ihrer räumlichen Dimensionen untereinander unterscheiden.

**13.** System nach einem der Ansprüche 8 bis 12, **dadurch gekennzeichnet, dass** das System weiterhin eine Röntgeneinrichtung (20, 30) aufweist, die dazu konfiguriert ist, ein 2D-Röntgenbild des Körpers (1) zu erzeugen, sowie ein Analysemittel, das dazu konfiguriert ist, die zweidimensionalen Positionen der Röntgenmarker (10) in einem Bildkoordinatensystem (K') des 2D-Röntgenbildes (B) zu bestimmen, und das weiterhin dazu konfiguriert ist, mögliche 3D-Ortskoordinaten (M) jedes Röntgenmarkers (10) in dem 3D-Weltkoordinatensystem (K) als Strahlen zu bestimmen, die jeweils von einem Ursprung, der der Position einer Strahlenquelle (20) der Röntgeneinrichtung zur Erzeugung des 2D-Röntgenbildes (B) entspricht, zur Position des jeweiligen Röntgenmarkers (10) in einer Bildkoordinatenebene (B') verlaufen, und das ferner dazu konfiguriert ist, den räumlichen Verlaufs des Körpers (1) unter Verwendung der besagten möglichen 3D-Ortskoordinaten (M) iterativ zu bestimmen.

**14.** System nach Anspruch 13, **dadurch gekennzeichnet, dass** das Analysemittel weiterhin dazu konfiguriert ist, zur besagte iterative Bestimmung:

(a) an vorgegebene Startwerten (E) für die 3D-Ortskoordinaten aus der Menge der möglichen 3D-Ortskoordinaten (M) eine 3D-Kurve anzufitten,

(b) zum Erhalt aktualisierter 3D-Ortskoordinaten (O) zumindest eine 3D-Ortskoordinate der 3D-Kurve entlang des zugeordneten Strahls (M) auf eine mögliche weitere 3D-Ortskoordinate (O') zu verschieben, um aktualisierte Ortskoordinaten (O) zu erhalten,

(c) eine 3D-Kurve (C) an die aktualisierten 3D-Ortskoordinaten (O) anzufitten,

(d) eine Rückprojektion der 3D-Kurve (C) in das Bildkoordinatensystem (K') vorzunehmen und jene Rückprojektion mit dem 2D-Röntgenbild (B) zu vergleichen, und

(e) die iterative Bestimmung beginnend mit Schritt (b) bis zum Erreichen eines vordefinierten Kriteriums fortzusetzen.

**15.** System nach Anspruch 14, **dadurch gekennzeichnet, dass** das Analysemittel dazu ausgebildet ist, im Schritt (b) die weitere Ortskoordinate (O') so zu wählen, dass sie auf dem der weiteren Ortskoordinate (O') zugeordneten Strahl (M) sowie in einer Kugelschale (S) um eine 3D-Ortskoordinate eines benachbarten Röntgenmarkers (10) liegt, wobei der äußere Radius ($R_{außen}$) der Kugelschale (S) durch den Abstand (D) der beiden benachbarten Röntgenmarker (10) entlang des Körpers (1) gegeben ist, und wobei der innere Radius ($R_{innen}$) der Kugelschale (S) durch die Länge einer zwischen den beiden Röntgenmarkern (10) erstreckte Bogensehne (S') bei maximaler Krümmung des Körpers (1) zwischen den beiden benachbarten Röntgenmarkern (10) gegeben ist.

Fig. 1

20

10

30

A

Abstand D senkrecht zur Detektorebene

Abstand Quelle-Detektor

Fig. 2

1. Segmentierung der Elektrode insbesondere der Röntgenmarker nach Schwellenwertverfahrung (bzw. Mustererkennung)

2. Bestimmung der Koordinaten der Röntgenmarker im Bildkoordinatensystem (Massenschwerpunkte)

3. Berechnung der möglichen 3D Ortskoordinaten der Röntgenmarker entlang der Strahlgeometrie mit der Strahlenquelle als Ursprung (je Röntgenmarker eine Gerade mit Raum)

4. Anpassung einer 3D-Kurve durch einen (beliebigen) Startwert der möglichen 3D-Ortskoordinaten, z.B. auf der Ebene mittig zwischen Strahlenquelle und Detektor parallel zum Detektor (je Röntgenmarker ein Punkt im Raum)

5. Rekursive Verschiebung der Ortskoordinaten der 3D-Kurve entlang der Strahlengänge mit Hilfe der physikalischen Einschränkungen:
- Abstand der Röntgenmarker entlang der Elektrode
- Maximaler Biegeradius einer Elektrode (>> halber Elektrodendurchmesser)
- Rückprojektion der angenommenen 3D-Koordinaten auf 2D-Bildkoordinaten
- Vergleich der Rückprojektion mit abgebildeter Elektrode, insbesondere Größe, Orientierung und Position der Röntgenmarker im Röntgenbild

## Fig. 3

EP 3 142 073 A1

Fig. 4

Fig. 5

**Europäisches Patentamt**
**European Patent Office**
**Office européen des brevets**

# EUROPÄISCHER RECHERCHENBERICHT

Nummer der Anmeldung

EP 16 18 3384

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 01/30254 A1 (CEDARA SOFTWARE CORP [CA]; MENHARDT WIDO [US]) 3. Mai 2001 (2001-05-03) | 1,3,6,7, 13-15 | INV. G06T7/73 A61B6/12 |
| Y | * Seite 3, Zeile 13 * * Abbildung 3 * * Seite 8, Zeile 32 * * Anspruch 9 * * Abbildung 2 * * Seite 3, Zeile 19 - Zeile 20 * * Seite 7, Zeilen 14-18,25 * * Seite 8, Zeile 16 - Zeile 28 * ----- | 2,4,5,8 | |
| Y | WO 2011/095927 A1 (KONINKL PHILIPS ELECTRONICS NV [NL]; PHILIPS INTELLECTUAL PROPERTY [DE]) 11. August 2011 (2011-08-11) | 4,5,8 | |
| A | * Seite 20, Zeile 32 - Zeile 34 * * Anspruch 6 * * Abbildung 2 * ----- | 3,7 | |
| Y | US 2010/135553 A1 (JOGLEKAR AJINKYA M [US]) 3. Juni 2010 (2010-06-03) * Absatz [0006] * ----- | 2 | |
| X | DE 10 2012 207261 A1 (SIEMENS AG [DE]) 7. November 2013 (2013-11-07) * Absätze [0002], [0003], [0007], [0008] * * Abbildung 4 * ----- | 9-12 | RECHERCHIERTE SACHGEBIETE (IPC) G06T A61B |
| A | US 2008/262342 A1 (AVERBRUCH DORIAN [IL]) 23. Oktober 2008 (2008-10-23) * Absätze [0059], [0060] * ----- | 10,11 | |
| A | WO 2013/169814 A1 (ANGIOMETRIX CORP [US]) 14. November 2013 (2013-11-14) * Seite 7, letzter Zeile * ----- | 12 | |

Der vorliegende Recherchenbericht wurde für alle Patentansprüche erstellt

| Recherchenort | Abschlußdatum der Recherche | Prüfer |
|---|---|---|
| München | 17. Januar 2017 | Winkler, Gregor |

EPO FORM 1503 03.82 (P04C03)

ANHANG ZUM EUROPÄISCHEN RECHERCHENBERICHT
ÜBER DIE EUROPÄISCHE PATENTANMELDUNG NR.

EP 3 142 073 A1

EP 16 18 3384

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

17-01-2017

| Im Recherchenbericht angeführtes Patentdokument | Datum der Veröffentlichung | Mitglied(er) der Patentfamilie | Datum der Veröffentlichung |
|---|---|---|---|
| WO 0130254 A1 | 03-05-2001 | AU 1013001 A<br>WO 0130254 A1 | 08-05-2001<br>03-05-2001 |
| WO 2011095927 A1 | 11-08-2011 | EP 2531108 A1<br>RU 2012137493 A<br>US 2012302876 A1<br>WO 2011095927 A1 | 12-12-2012<br>10-03-2014<br>29-11-2012<br>11-08-2011 |
| US 2010135553 A1 | 03-06-2010 | KEINE | |
| DE 102012207261 A1 | 07-11-2013 | DE 102012207261 A1<br>US 2013293535 A1 | 07-11-2013<br>07-11-2013 |
| US 2008262342 A1 | 23-10-2008 | EP 2140426 A2<br>JP 2010522597 A<br>US 2008262342 A1<br>US 2016183888 A1<br>WO 2009024852 A2 | 06-01-2010<br>08-07-2010<br>23-10-2008<br>30-06-2016<br>26-02-2009 |
| WO 2013169814 A1 | 14-11-2013 | AU 2013259659 A1<br>CA 2873035 A1<br>CN 104519793 A<br>EP 2846688 A1<br>JP 2015515913 A<br>US 2015245882 A1<br>WO 2013169814 A1 | 15-01-2015<br>14-11-2013<br>15-04-2015<br>18-03-2015<br>04-06-2015<br>03-09-2015<br>14-11-2013 |

EPO FORM P0461

Für nähere Einzelheiten zu diesem Anhang : siehe Amtsblatt des Europäischen Patentamts, Nr.12/82

16